(19) Europäisches Patentamt
European Patent Office
Office européen des brevets

(11) EP 1 420 790 B1

(12) EUROPEAN PATENT SPECIFICATION

(45) Date of publication and mention of the grant of the patent: **21.09.2005 Bulletin 2005/38**

(21) Application number: **02794772.0**

(22) Date of filing: **10.08.2002**

(51) Int Cl.[7]: **A61K 31/517**, A61K 31/422, A61K 31/4045, A61K 31/48, A61P 29/00, A61P 25/06 // A61K31:517, A61K31:422, A61K31:517, A61K31:4045, A61K31:517, A61K31:48

(86) International application number: **PCT/EP2002/008993**

(87) International publication number: **WO 2003/015787 (27.02.2003 Gazette 2003/09)**

(54) **USE OF BIBN4096 IN COMBINATION WITH OTHER ANTIMIGRAINE DRUGS FOR THE TREATMENT OF MIGRAINE**

VERWENDUNG VON BIBN4096 IN KOMBINATION MIT ANDEREN MIGRÄNEMITTELN ZUR BEHANDLUNG VON MIGRÄNE

UTILISATION DE BIBN4096 EN COMBINAISON AVEC D'AUTRES MEDICAMENTS CONTRE LA MIGRAINE POUR LE TRAITEMENT DE LA MIGRAINE

(84) Designated Contracting States:
**AT BE BG CH CY CZ DE DK EE ES FI FR GB GR IE IT LI LU MC NL PT SE SK TR**
Designated Extension States:
**LT LV RO SI**

(30) Priority: **17.08.2001 DE 10139410**

(43) Date of publication of application: **26.05.2004 Bulletin 2004/22**

(73) Proprietor: **Boehringer Ingelheim Pharma GmbH & Co.KG**
**55216 Ingelheim am Rhein (DE)**

(72) Inventors:
- **DOODS, Henri**
  **88447 Warthausen (DE)**
- **RUDOLF, Klaus**
  **88447 Warthausen (DE)**
- **EBERLEIN, Wolfgang**
  **88400 Biberach (DE)**

(56) References cited:
**WO-A-01/10425** **WO-A-98/11128**

- **DOODS H ET AL: "PHARMACOLOGICAL PROFILE OF BIBN4096BS, THE FIRST SELECTIVE SMALL MOLECULE CGRP ANTAGONIST" BRITISH JOURNAL OF PHARMACOLOGY, BASINGSTOKE, HANTS, GB, vol. 129, no. 3, 2000, pages 420-423, XP000992559 ISSN: 0007-1188**
- **FERRARI M D ET AL: "Oral triptans (serotonin 5-HT1B/1D agonists) in acute migraine treatment: a meta-analysis of 53 trials" LANCET, XX, XX, vol. 358, no. 9294, 17 November 2001 (2001-11-17), pages 1668-1675, XP004323637 ISSN: 0140-6736**
- **DATABASE MEDLINE [Online] 15 November 1993 (1993-11-15) HOERNECKE R ET AL: "[Treatment of migraine attacks: combination of dihydroergotamine tartrate and paracetamol in comparison with individual drugs and placebo]" Database accession no. NLM8295604 XP002199881 & MEDIZINISCHE KLINIK (MUNICH, GERMANY: 1983) GERMANY 15 NOV 1993, vol. 88, no. 11, 15 November 1993 (1993-11-15), pages 642-648, ISSN: 0723-5003**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

• DATABASE MEDLINE [Online] January 1992 (1992-01) SAADAH H A: "Abortive headache therapy with intramuscular dihydroergotamine." Database accession no. NLM1555927 XP002216638 & HEADACHE. UNITED STATES JAN 1992, vol. 32, no. 1, January 1992 (1992-01), pages 18-20, ISSN: 0017-8748

## Description

### Field of the invention

Migraine is one of the most common neurological disorders, involving periodical attacks of headache and nausea as well as a plethora of other symptoms. Although considerable progress has been made, the pathophysiology of migraine is still not understood. However, several observations point to an involvement of Calcitonin Gene-Related Peptide (CGRP). Migraine headache involves the activation of the trigeminal system and dilatation of cranial vessels. CGRP is localized to neurons in the trigeminal ganglia and CGRP levels are increased during a migraine attack, presumably causing the vasodilation observed. Accordingly, it is conceivable that inhibition of CGRP-evoked dilatation of the cranial vessels may provide a novel treatment for migraine headache.

Widely used antimigraine drugs are the so-called "triptans", e.g. sumatriptan and zolmitriptan. These compounds elicit their antimigraine effects due to their vasoconstrictive properties and presumably their inhibition of the release of the neuropeptide calcitonin gene related peptide (CGRP) (Ferrari, M. D., Saxena, P. R. (1995), 5-$HT_1$ receptors in migraine pathophysiology and treatment, *Eur. J. Neurology*, **2**, 5-21; Johnson, K. W., Phebus, L. A., Cohen, M. L. (1998), Serotonin in migraine: Theiroes, animal models and emerging therapies, *Progress in Drug Research,* Vol. **51,** 220-244), the levels of which are assumed to be increased during a migraine attack (Edvinsson, L., Goadsby, P. J. (1994), Neuropeptides in migraine and duster headache, *Cephalgia*, **14**(5), 320-327). A completely novel approach to treat migraine is the use of CGRP antagonists (Doods, H., Hallermayer, G., Wu, D., Entzeroth, M., Rudolf, K., Engel, W., Eberlein, W. (2000), Pharmacological profile of BIBN4096BS, the first selective small molecule CGRP antagonist, *Br. J. Pharmacol.*, **129,** 420-423).

### Background of the Invention

WO 98/11128 discloses modified amino acids having CGRP-antagonistic properties, their use and methods for their preparation as well as their use for the production and purification of antibodies and as labelled compounds in RIA and ELISA assays and as diagnostic or analytic auxiliary agents in neurotransmitter research. In view of their pharmacological properties the modified amino acids are thus suitable for acute and prophylactic treatment of headache, particularly migraine and cluster headaches.

### Summary of the Invention

Unexpectedly, it was found that in a model which is considered to predict the antimigraine effects of drugs the combination of two drugs with a completely different mode of action, namely specified a 5-$HT_{1B/1D}$ agonist or an ergot alkaloid and the CGRP antagonist disclosed in the WO 98/11128 A1, namely

A = 1-[$N^2$-[3,5-Dibromo-N-[[4-(3,4-dihydro-2(1H)-oxochinazolin-3-yl)-1-piperidinyl]-carbonyl]-D-tyrosyl]-L-lysyl]-4-(4-pyridinyl)-piperazine [BIBN4096BS],

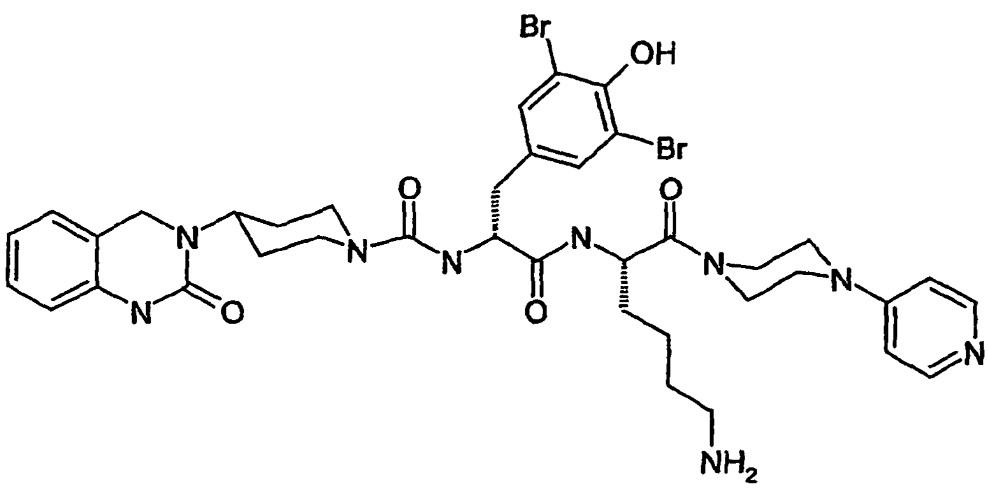

results in a highly significant better effect compared to the effect of one drug alone.

## Detailed Description of the Invention

As a first aspect the present invention provides a composition for treatment or prevention of indications selected from the group consisting of headache, migraine and cluster headaches, comprising a therapeutically effective amount of BIBN4096BS or a physiologically acceptable salt thereof and a therapeutically effective amount of another active antimigraine drug (A) selected from sumatriptan, zolmitriptan and dihydroergotamin to a person in need of such treatment.

As a preferred embodiment in the composition according to the invention drug (A) is selected from sumatriptan or the physiologically acceptable salts thereof.

The dosage for the combined migraine drug (A) is appropriately 1/50 of the lowest dose normally recommended up to 1/1 of the normally recommended dosage, preferably 1/50 to 1/6 and more preferably 1/20 to 1/10, by orally, nasally, subcutaneous or intravenous route. The normally recommended dose for the combined migraine drug (A) should be understood to be the dose disclosed in Rote Liste Win® 2001/l, Editio Cantor Verlag Aulendorf.

According to the invention BIBN4096BS or a physiologically acceptable salt thereof may be administered by intravenous or subcutaneous route in a dosage of 0.0001 to 3 mg/kg of body weight or by oral, nasal or inhalative route in a dosage of 0.1 to 10 mg/kg of body weight once, twice or trice a day, in combination with
sumatriptan or a physiologically acceptable salt thereof which may be administered by oral route in a dosage of 0.03 to 1.43 mg/kg of body weight once, twice or trice a day or
by intravenous or subcutaneous route in a dosage of 0.002 to 0.09 mg/kg of body weight once or twice a day or
by rectal route in a dosage of 0.007 to 0.36 mg/kg of body weight once or twice a day or
by nasal route in a dosage of 0.006 to 0.29 mg/kg of body weight once or twice a day or in combination with
Zolmitriptan or a physiologically acceptable salt thereof which may be administered by oral route in a dosage of 0.0007 to 0.036 mg/kg of body weight once or twice a day or
in combination with dihydroergotamine or a physiologically acceptable salt thereof which may be administered by oral route in a dosage of 0.001 to 0.07 mg/kg of body weight once or twice a day or
In a preferred embodiment of the invention BIBN4096BS or a physiologically acceptable salt thereof may be administered by intravenous or subcutaneous route in a dosage of 0.0001 to 3 mg/kg of body weight or by oral, nasal or inhalative route in a dosage of 0.1 to 10 mg/kg of body weight once, twice or trice a day, in combination with
sumatriptan or a physiologically acceptable salt thereof which may be administered by oral route in a dosage of 0.03 to 0.24 mg/kg of body weight once, twice or trice a day or
by intravenous or subcutaneous route in a dosage of 0.002 to 0.015 mg/kg of body weight once or twice a day or
by rectal route in a dosage of 0.007 to 0.06 mg/kg of body weight once or twice a day or
by nasal route in a dosage of 0.006 to 0.048 mg/kg of body weight once or twice a day or
in combination with Zolmitriptan or a physiologically acceptable salt thereof which may be administered by oral route in a dosage of 0.0007 to 0.006 mg/kg of body weight once or twice a day or
in combination with dihydroergotamine or a physiologically acceptable salt thereof which may be administered by oral route in a dosage of 0.001 to 0.01 mg/kg of body weight once or twice a day or
In a more preferred embodiment of the invention SISN40968S or a physiologically acceptable salt thereof may be administered by intravenous or subcutaneous route in a dosage of 0.0001 to 3 mg/kg of body weight or by oral, nasal or inhalative route in a dosage of 0.1 to 10 mg/kg of body weight once, twice or trice a day, in combination with
sumatriptan or a physiologically acceptable salt thereof which may be administered by oral route in a dosage of 0.075 to 0.143 mg/kg of body weight once, twice or trice a day or
by intravenous or subcutaneous route in a dosage of 0.005 to 0.009 mg/kg of body weight once or twice a day or
by rectal route in a dosage of 0.0175 to 0.036 mg/kg of body weight once or twice a day or
by nasal route in a dosage of 0.015 to 0.029 mg/kg of body weight once or twice a day or
in combination with Zolmitriptan or a physiologically acceptable salt thereof which may be administered by oral route in a dosage of 0.00175 to 0.0036 mg/kg of body weight once or twice a day or
in combination with dihydroergotamine or a physiologically acceptable salt thereof which may be administered by oral route in a dosage of 0.0025 to 0.007 mg/kg of body weight once or twice a day or

The present invention provides a pharmaceutical composition for treating or preventing headache, migraine or cluster headaches comprising a therapeutically effective amount of BIBN4096BS or a physiologically acceptable salt thereof and an antimigraine drug (A) selected from the group consisting of sumatriptan, zolmitriptan and dihydroergotamin or a physiologically acceptable salt thereof as a combined preparation for simultaneous or sequential administration.

A pharmaceutical composition according to the invention may comprise a single dosage unit of 0.1 to 10 mg of BIBN4096BS and
a single dosage unit of 1 to 100 mg of sumatriptan or
a single dosage unit of 0.1 to 2.5 mg of zolmitriptan or

a single dosage unit of 0.1 to 5 mg of dihydroergotamin or
a single dosage unit of 7.5 to 15 mg of meloxicam.

All doses or dosage units of a physiologically acceptable salt of an active compound mentioned hereinbefore should be understood as the dose or dosage of the active compound itself.

Furthermore, a pharmaceutical composition according to the invention may be a kit of parts for treating or preventing headache, migraine or cluster headaches, which kit comprises

(a) a first containment containing a pharmaceutical composition comprising a therapeutically effective amount of BIBN4096BS or a physiologically acceptable salt thereof and one or more pharmaceutically acceptable diluents and/or carriers; and

(b) a second containment containing a pharmaceutical composition comprising sumatriptan, zolmitriptan or dihydroergotamin or a physiologically acceptable salt thereof and one or more pharmaceutically acceptable diluents and/or carriers.

A preferred kit of parts comprises sumatriptan in the second containment.

A further aspect of the present invention is the use of BIBN4096BS or a physiologically acceptable salt thereof in combination with another active antimigraine drug (A) as defined above for the manufacture of a pharmaceutical composition for treating or preventing headache, migraine or cluster headaches. Drug (A) and preferred embodiments thereof as well as pharmaceutical compositions are mentioned hereinbefore. Most preferred with respect to all aspects of the invention is the combination of BIBN4096BS with sumatriptan or of physiologically acceptable salts thereof.

Several of the drug (A) components mentioned hereinbefore are already on the market, e.g. sumatriptan is sold under the trade name imigran®, zolmitriptan is sold under the trade name ascotop® and dihydroergotamin and the pharmaceutically acceptable salts thereof under the trade name agit®.

BIBN4096BS can be administered using for instance pharmaceutical formulations disclosed in WO 98/11128 or using one of the following pharmaceutical formulations:

capsules for powder inhalation containing 1 mg of active substance,

inhalable solution for nebulisers containing 1 mg of active substance,

propellant gas-operated metering aerosol containing 1 mg of active substance,

nasal spray containing 1 mg of active substance,

tablets containing 20 mg of active substance,

capsules containing 20 mg of active substance,

aqueous solution for nasal application containing 10 mg of active substance,

aqueous solution for nasal application containing 5 mg of active substance, or

suspension for nasal application containing 20 mg of active substance.

Example 1

In order to examine the pharmacological activity of combinations according to the invention the following experiments have been carried out.

Measurement of facial skin blood flow

Facial skin blood flow was measured by a modified method described by Escott et al. (Escott, K. J., Beattie, D. T., Connor, H. E., Brain, S. D. (1995), Trigeminal ganglion stimulation increases facial skin blood flow in the rat a major role for calcitonin gene-related peptide, *Brain Research,* **669**(1), 93-99). Fasted male wistar rats (strain CHbb: THOM, 280-320g) were anaesthetized with sodium pentobarbitone (initially with 60 mg/kg i.p. and maintained throughout the experiment with an intraperitoneal infusion of 30 mg/kg/h through a 23 G needle using a solution of 10 mg/ml). Both sides of the buccal area of the facial skin were shaved and depilated with a commercial depilatory cream (Pilca, Schwarzkopf & Henkel, 40551 Düsseldorf, Germany). The trachea was cannulated and the animals were artificially

respired (80 strokes/min) with room air supplemented with oxygen. The body temperature was maintained at 37°C by an automated heating pad. The left femoral artery and the left femoral vein were cannulated for the continuous measurement of arterial blood pressure and intravenous administration of test compounds, respectively. Neuromuscular blockade was achieved by intravenous administration of pancuronium bromide (1mg/kg/0.5ml, 5 min prior to each electrical stimulation). Heart rate was derived from the blood pressure signal. Blood pressure and heart rate were continuously monitored throughout the course of the experiment to assess the level of anaesthesia and to monitor the cardiovascular effects of the drugs used in this study.

The animals were placed in a stereotaxic frame and a longitudinal incision was made in the scalp. A small hole was drilled in the skull (left or right) and a bipolar electrode (Rhodes SNEX-100 supplied by David Kopf Instruments, Tujunga, 91042 Califomia, U.S.A.) was lowered using a micromanlpulator, into the trigeminal ganglion (0.32 cm dorsal to bregma, ± 0.30 cm lateral from the midline and 0.95 cm below the dural surface). The position of the electrodes in the trigeminal ganglia were checked visually at the end of each experiment following removal of the brain. The trigeminal ganglion was stimulated at 10 Hz, 1 mA, 1 msec for 30 seconds using a stimulator supplied by Hugo Sachs Elektronik (79232 March-Hugstetten, Germany). Microvascular blood flow changes in the facial skin were measured by Laser Doppler flowmetry with a Periflux laser doppler system (PeriFLUX 4001, wave length 780 nM; time constant 3 s, Perimed AB, Järfälla, S-17526, Sweden). Standard laser doppler probes (PROBE 408) were positioned on either side of the face approximately 0.5 cm below the centre of the eye, an area innervated by the maxillary branch (V2) of the trigeminal nerve. Blood flow changes were measured as flux in arbitrary units and expressed as area under the flux curve ($mm^2$) according to Escott et al. (1995).

Experimental protocol

After 30 min of equilibration, the animals were subjected to three periods of electrical stimulation, separated by a 30 min interval. The first stimulation was used as a control for the subsequent stimulations. Saline, single compound or the combination were administered intravenously 5 min prior to the second stimulation.

The results are given in the following table 1. They show that the improved potency of the combination of 5-$HT_{1B/1D}$ agonists or other antimigraine drugs in general with a CGRP antagonist would allow higher efficacy, would allow lower doses of each compound resulting in similar efficacy with less side effects and the addition of the two mechanisms might result in less headache recurrence.

Table 1:

Effect of BIBN 4096 BS in combination with other antimigraine drugs on facial skin vasodilation induced by electrical trigeminal ganglion stimulation in the rat.

| treatment | % of trigeminus stimulation | n | % inhibition compared to control value |
|---|---|---|---|
| | | | |
| saline (control) | 82.7 ± 4.4 | 11 | - |
| | | | |
| BIBN 4096 BS (0.03 mg/kg) | 60.3 ± 5.1 | 8 | 27.1 |
| | | | |
| Sumatriptan (1.0 mg/kg) | 68.8 ± 6.8 | 7 | 16.8 |
| BIBN 4096 BS + Sumatriptan | 26.6 ± 5.4[a] | 6 | 67.8 |
| (0.03 mg + 1.0 mg)/kg | | | |
| | | | |
| Zolmitriptan (0.1 mg/kg) | 55.6 ± 4.8 | 6 | 32.8 |
| BIBN 4096 BS + Zolmitriptan | 27.3 ± 6.0[b] | 6 | 67.0 |
| (0.03 mg + 0.1 mg)/kg | | | |
| | | | |

[a] significant, $p < 0.001$, compared to Sumatriptan

[b] significant, $p < 0.01$, compared to Zolmitriptan

Table 1: (continued)

| Effect of BIBN 4096 BS in combination with other antimigraine drugs on facial skin vasodilation induced by electrical trigeminal ganglion stimulation in the rat. | | | |
|---|---|---|---|
| treatment | % of trigeminus stimulation | n | % inhibition compared to control value |
| DHE (0.1 mg/kg) | 60.4 ± 4.1 | 6 | 27.0 |
| BIBN 4096 BS + DHE | 20.9 ± 3.1[c] | 6 | 74.7 |
| (0.03 mg + 0.1 mg)/kg | | | |
| | | | |
| DHE = Dihydroergotamin | | | |

[c] significant, $p < 0.001$, compared to DHE

The Examples which follow describe pharmaceutical preparations which contain as active substance BIBN4096BS or a pharmaceutically acceptable salt thereof:

Example 2

Capsules for powder inhalation with 1 mg of active substance

| Composition: | |
|---|---|
| 1 capsule for powder inhalation contains: | |
| active substance | 1.0 mg |
| lactose | 20.0 mg |
| hard gelatine capsules | 50.0 mg |
| | 71.0 mg |

Method of preparation:

The active substance is ground to the particle size needed for inhalation. The ground active substance is homogeneously mixed with the lactose. The mixture is packed into hard gelatine capsules.

Example 3

Inhalable solution for Respimat® with 1 mg of active substance

| Composition: | |
|---|---|
| 1 spray contains: | |
| active substance | 1.0 mg |
| benzalkonium chloride | 0.002 mg |
| disodium edetate | 0.0075 mg |
| purified water ad | 15.0 µl |

Method of preparation:

The active substance and benzalkonium chloride are dissolved in water and packed in Respimat® cartridges.

Example 4

Inhalable solution for nebulisers with 1 mg of active substance

| Composition: | |
|---|---|
| 1 vial contains: | |
| active substance | 0.1 g |
| sodium chloride | 0.18 g |
| benzalkonium chloride | 0.002 g |
| purified water ad | 20.0 ml |

Method of preparation:

Active substance, sodium chloride and benzalkonium chloride are dissolved in water.

Example 5

Propellant gas-operated metering aerosol with 1 mg of active substance

| Composition: | |
|---|---|
| 1 spray contains: | |
| active substance | 1.0 mg |
| lecithin | 0.1 % |
| propellant gas ad | 50.0 µl |

Method of preparation:

The micronised active substance is homogeneously suspended in the mixture of lecithin and propellant gas. The suspension is transferred into a pressurised container with a metering valve.

Example 6

Nasal spray with 1 mg of active substance

| Composition: | |
|---|---|
| 1 spray jet contains | |
| active substance | 1.0 mg |
| mannitol | 5.0 mg |
| disodium edetate | 0.05 mg |
| ascorbic acid | 1.0 mg |
| purified water ad | 0.1 ml |

Method of preparation:

The active substance and the excipients are dissolved in water and transferred into a suitable container.

Example 7

Injectable solution with 5 mg of active substance per 5 ml

| Composition: | |
|---|---|
| active substance in basic form | 5 mg |
| acid/salt-forming agent in the amount needed to form a neutral salt | q.s. |
| glucose | 250 mg |
| human serum albumin | 10 mg |
| glycofurol | 250 mg |
| water for injections ad | 5 ml |

Preparation:

Dissolve the glycofurol and glucose in water for injections (Wfl); add human serum albumin; add salt-forming agent; dissolve active substance with heating; make up to specified volume with Wfl; transfer into ampoules under nitrogen gas.

Example 8

Injectable solution for subcutaneous administration containing 5 mg of active substance per 1 ml

| Composition: | |
|---|---|
| active substance | 5 mg |
| glucose | 50 mg |
| polysorbate 80 = Tween 80 | 2 mg |
| water for injections ad | 1 ml |

Preparation:

Dissolve glucose and polysorbate in water for injections; dissolve active substance with heating or using ultrasound; make up to specified volume with Wfl; transfer into ampoules under inert gas.

Example 9

Injectable solution containing 100 mg of active substance per 10 ml

| Composition: | |
|---|---|
| active substance | 100 mg |
| monopotassium dihydrogen phosphate = $KH_2PO_4$ | 12 mg |
| disodium hydrogen phosphate = $Na_2HPO_4 \cdot 2H_2O$ | 2 mg |
| sodium chloride | 180 mg |
| human serum albumin | 50 mg |
| polysorbate 80 | 20 mg |
| water for injections ad | 10 ml |

Preparation:

Dissolve polysorbate 80, sodium chloride, monopotassium dihydrogen phosphate and disodium hydrogen phosphate in water for injections (Wfl); add human serum albumin; dissolve active substance with heating; make up to specified volume with Wfl; transfer into ampoules.

Example 10

Lyophilisate containing 10 mg of active substance

| Composition: | |
|---|---|
| active substance in basic form | 10 mg |
| acid/salt-forming agent in the amount needed to form a neutral salt | q.s. |
| mannitol | 300 mg |
| water for injections ad | 2 ml |

Preparation:

Dissolve mannitol in water for injections (Wfl); add salt-forming agent; dissolve active substance with heating; make up to specified volume with Wfl; transfer into vials; freeze-dry.

| Solvent for lyophilisate: | |
|---|---|
| polysorbate 80 = Tween 80 | 20 mg |
| mannitol | 200 mg |
| water for injections ad | 10 ml |

Preparation:

Dissolve polysorbate 80 and mannitol in water for injections (Wfl); transfer into ampoules.

Example 11

Lyophilisate containing 5 mg of active substance

| Composition: | |
|---|---|
| active substance in basic form | 5 mg |
| polar or nonpolar solvent (which can be removed by freeze-drying) ad | 1 ml |

Preparation:

Dissolve active substance in suitable solvent; transfer into vials; freeze-dry.

| Solvent for lyophilisate: | |
|---|---|
| polysorbate 80 = Tween 80 | 5 mg |
| mannitol | 100 mg |
| water for injections ad | 2 ml |

Preparation:

Dissolve polysorbate 80 and mannitol in water for injections (Wfl); transfer into ampoules.

Example 12

Tablets containing 20 mg of active substance

| Composition: | |
|---|---|
| active substance | 20 mg |
| lactose | 120 mg |
| maize starch | 40 mg |
| magnesium stearate | 2 mg |
| Povidone K 25 | 18 mg |

Preparation:

Homogeneously mix the active substance, lactose and maize starch; granulate with an aqueous solution of Povidone; mix with magnesium stearate; press in a tablet press; weight of tablet 200 mg.

Example 13

Capsules containing 20 mg of active substance

| Composition: | |
|---|---|
| active substance | 20 mg |
| maize starch | 80 mg |
| highly dispersed silica | 5 mg |
| magnesium stearate | 2.5 mg |

Preparation:

Homogeneously mix the active substance, maize starch and silica; mix with magnesium stearate; transfer mixture into size 3 hard gelatine capsules in a capsule filling machine.

Example 14

Suppositories containing 50 mg of active substance

| Composition: | |
|---|---|
| active substance | 50 mg |
| hard fat (Adeps solidus) q.s. ad | 1700 mg |

Preparation:

Melt the hard fat at about 38°C; homogeneously disperse the ground active substance in the molten hard fat; after cooling to about 35°C, pour into chilled moulds.

Example 15

Aqueous solution for nasal administration containing 10 mg of active substance

| Composition: | |
|---|---|
| active substance | 10.0 mg |
| hydrochloric acid in the amount needed to form a neutral salt | |
| methyl parahydroxybenzoate (PHB) | 0.01 mg |
| propyl parahydroxybenzoate (PHB) | 0.005 mg |
| purified water ad | 1.0 ml |

Preparation:

The active substance is dissolved in purified water; hydrochloric acid is added until the solution is clear; methyl and propyl PHB are added; the solution is made up to the specified volume with purified water; the solution is filtered sterile and transferred into a suitable container.

Example 16

Aqueous solution for nasal administration containing 5 mg of active substance

| Composition: | |
|---|---|
| active substance | 5 mg |
| 1,2-propanediol | 300 mg |
| hydroxyethylcellulose | 5 mg |
| sorbic acid | 1 mg |
| purified water ad | 1 ml |

Preparation:

The active substance is dissolved in 1,2-propanediol; a hydroxyethyl-cellulose solution in purified water containing sorbic acid is prepared and added to the solution of active substance; the solution is filtered sterile and transferred into a suitable container.

Example 17

Aqueous solution for intravenous administration containing 5 mg of active substance

| Composition: | |
|---|---|
| active substance | 5 mg |
| 1,2-propanediol | 300 mg |
| mannitol | 50 mg |
| water for injections (Wfl) ad | 1 ml |

Preparation:

The active substance is dissolved in 1,2-propanediol; the solution is made up to approximately the specified volume with Wfl; the mannitol is added and made up to approximately the specified volume with Wfl; the solution is

filtered sterile, transferred into individual containers and autoclaved.

Example 18

Liposomal formulation for intravenous injection containing 7.5 mg of active substance

| Composition: | |
|---|---|
| active substance | 7.5 mg |
| egg lecithin, e.g. Lipoid E 80 | 100.0 mg |
| cholesterol | 50.0 mg |
| glycerol | 50.0 mg |
| water for injections ad | 1.0 ml |

Preparation:

The active substance is dissolved in a mixture of lecithin and cholesterol; the solution is added to a mixture of glycerol and Wfl and homogenised by high pressure homogenisation or by the Microfluidizer technique; the liposomal formulation obtained is transferred into a suitable container under aseptic conditions.

Example 19

Suspension for nasal administration containing 20 mg of active substance

| Composition: | |
|---|---|
| active substance | 20.0 mg |
| carboxymethylcellulose (CMC) | 20.0 mg |
| sodium monohydrogen phosphate/sodium dihydrogen phosphate buffer pH 6.8 | q.s. |
| sodium chloride | 8.0 mg |
| methyl parahydroxybenzoate | 0.01 mg |
| propyl parahydroxybenzoate | 0.003 mg |
| purified water ad | 1.0 ml |

Preparation:

The active substance is suspended in an aqueous CMC solution; the other ingredients are added successively to the suspension and the suspension is topped up to the specified volume with purified water.

Example 20

Aqueous solution for subcutaneous administration with 10 mg of active substance

| Composition: | |
|---|---|
| active substance | 10.0 mg |
| sodium monohydrogen phosphate/sodium dihydrogen phosphate buffer q.s. ad pH | 7.0 |
| sodium chloride | 4.0 mg |
| water for injections ad | 0.5 ml |

Preparation:

The active substance is dissolved in the phosphate buffer solution, after the addition of the common salt the solution is made up to the specified volume with water. The solution is filtered sterile, transferred into a suitable container and autoclaved.

Example 21

Aqueous suspension for subcutaneous administration containing 5 mg of active substance

| Composition: | |
|---|---|
| active substance | 5.0 mg |
| polysorbate 80 | 0.5 mg |
| water for injections | 0.5 ml |

Preparation:

The active substance is suspended in the polysorbate 80 solution and comminuted to a particle size of about 1 µm using a suitable dispersing technique (e.g. wet grinding, high pressure homogenisation, microfluidisation, etc.). The suspension is transferred into a corresponding container under aseptic conditions.

## Claims

1. A pharmaceutical composition for treating or preventing headache, migraine or cluster headaches comprising a therapeutically effective amount of BIBN4096BS or a physiologically acceptable salt thereof and an antimigraine drug (A) selected from sumatriptan, zolmitriptan and dihydroergotamin or a physiologically acceptable salt thereof as a combined preparation for simultaneous or sequential administration.

2. The pharmaceutical composition of claim 1 comprising a single dosage unit of 0.1 to 10 mg of BIBN4096BS and
a single dosage unit of 1 to 100 mg of sumatriptan or
a single dosage unit of 0.1 to 2.5 mg of zolmitriptan or
a single dosage unit of 0.1 to 5 mg of dihydroergotamin.

3. A kit of parts for treating or preventing headache, migraine or cluster headaches, which kit comprises

   (a) a first containment containing a pharmaceutical composition comprising a therapeutically effective amount of BIBN4096BS or a physiologically acceptable salt thereof and one or more pharmaceutically acceptable diluents and/or carriers; and
   (b) a second containment containing a pharmaceutical composition comprising sumatriptan, zolmitriptan or dihydroergotamin or a physiologically acceptable salt thereof and one or more pharmaceutically acceptable diluents and/or carriers.

4. The kit of parts according to claim 3, which kit comprises sumatriptan or a physiologically acceptable salt thereof in the second containment.

5. Use of BIBN4096BS or a physiologically acceptable salt thereof in combination with sumatriptan, zolmitriptan or dihydroergotamin or a physiologically acceptable salt thereof for the manufacture of a pharmaceutical composition for treating or preventing headache, migraine or cluster headaches.

6. The use according to claim 5, **characterized in that** BIBN4096BS or a physiologically acceptable salt thereof is to be administered by intravenous or subcutaneous route in a dosage of 0.0001 to 3 mg/kg of body weight or by oral, nasal or inhalative route in a dosage of 0.1 to 10 mg/kg of body weight once, twice or three times a day and sumatriptan or a physiologically acceptable salt thereof is administered by oral route in a dosage of 0.03 to 1.43 mg/kg of body weight once, twice or three times a day or

by intravenous or subcutaneous route in a dosage of 0.002 to 0.09 mg/kg of body weight once or twice a day or
by rectal route in a dosage of 0.007 to 0.36 mg/kg of body weight once or twice a day or
by nasal route in a dosage of 0.006 to 0.29 mg/kg of body weight once or twice a day or
Zolmitriptan or a physiologically acceptable salt thereof is to be administered by oral route in a dosage of 0.0007 to 0.036 mg/kg of body weight once or twice a day or
dihydroergotamine or a physiologically acceptable salt thereof is to be administered by oral route in a dosage of 0.001 to 0.07 mg/kg of body weight once or twice a day.

**7.** The use of BIBN4096BS or a physiologically acceptable salt thereof for manufacture of a pharmaceutical composition or a kit of parts according to any of claims 1 to 4.

**Patentansprüche**

**1.** Pharmazeutische Zusammensetzung zur Behandlung oder Verhütung von Kopfschmerzen, Migräne oder Cluster-Kopfschmerzen, umfassend eine therapeutisch wirksame Menge an BIBN4096BS oder eines physiologisch verträglichen Salzes desselben und einen Antimigräne-Arzneistoff (A), der ausgewählt ist aus Sumatriptan, Zolmitriptan und Dihydroergotamin oder einem physiologisch verträglichen Salz derselben, als kombiniertes Präparat für die gleichzeitige oder aufeinanderfolgende Verabreichung.

**2.** Pharmazeutische Zusammensetzung nach Anspruch 1, umfassend eine Einzeldosiseinheit von 0,1 bis 10 mg BIBN4096BS und
eine Einzeldosiseinheit von 1 bis 100 mg Sumatriptan oder
eine Einzeldosiseinheit von 0,1 bis 2,5 mg Zolmitriptan oder
eine Einzeldosiseinheit von 0,1 bis 5 mg Dihydroergotamin.

**3.** Kit zur Behandlung oder Verhütung von Kopfschmerzen, Migräne oder Cluster-Kopfschmerzen, welcher umfasst

(a) einen ersten Behälter, der eine pharmazeutische Zusammensetzung enthält, die eine therapeutisch wirksame Menge von BIBN4096BS oder eines physiologisch verträglichen Salzes desselben und ein(en) oder mehrere pharmazeutisch verträgliche Verdünnungsmittel und/oder Träger umfasst; und
(b) einen zweiten Behälter, der eine pharmazeutische Zusammensetzung enthält, die Sumatriptan, Zolmitriptan oder Dihydroergotamin oder ein physiologisch verträgliches Salz derselben und ein(en) oder mehrere pharmazeutisch verträgliche Verdünnungsmittel und/oder Träger umfasst.

**4.** Kit nach Anspruch 3, welcher Sumatriptan oder ein physiologisch verträgliches Salz desselben in dem zweiten Behälter umfasst.

**5.** Verwendung von BIBN4096BS oder eines physiologisch verträglichen Salzes desselben in Kombination mit Sumatriptan, Zolmitriptan oder Dihydroergotamin oder einem physiologisch verträglichen Salz derselben für die Herstellung einer pharmazeutischen Zusammensetzung zur Behandlung oder Verhütung von Kopfschmerzen, Migräne oder Cluster-Kopfschmerzen.

**6.** Verwendung nach Anspruch 5, **dadurch gekennzeichnet, dass** BIBN4096BS oder ein physiologisch verträgliches Salz desselben auf dem intravenösen oder subkutanen Weg in einer Dosis von 0,0001 bis 3 mg/kg Körpergewicht oder auf dem oralen, nasalen oder Inhalationsweg in einer Dosis von 0,1 bis 10 mg/kg Körpergewicht einmal, zweimal oder dreimal am Tag zu verabreichen ist und
Sumatriptan oder ein physiologisch verträgliches Salz desselben auf dem oralen Weg in einer Dosis von 0,03 bis 1,43 mg/kg Körpergewicht einmal, zweimal oder dreimal am Tag oder
auf dem intravenösen Weg oder subkutanen Weg in einer Dosis von 0,002 bis 0,09 mg/kg Körpergewicht einmal oder zweimal am Tag oder
auf dem rektalen Weg in einer Dosis von 0,007 bis 0,36 mg/kg Körpergewicht einmal oder zweimal am Tag oder
auf dem nasalen Weg in einer Dosis von 0,006 bis 0,29 mg/kg Körpergewicht einmal oder zweimal am Tag verabreicht wird oder
Zolmitriptan oder ein physiologisch verträgliches Salz desselben auf dem oralen Weg in einer Dosis von 0,0007 bis 0,036 mg/kg Körpergewicht einmal oder zweimal am Tag zu verabreichen ist oder
Dihydroergotamin oder ein physiologisch verträgliches Salz desselben auf dem oralen Weg in einer Dosis von

0,001 bis 0,07 mg/kg Körpergewicht einmal oder zweimal am Tag zu verabreichen ist.

7. Verwendung von BIBN4096BS oder eines physiologisch verträglichen Salzes desselben für die Herstellung einer pharmazeutischen Zusammensetzung oder eines Kits gemäß irgendeinem der Ansprüche 1 bis 4.

**Revendications**

1. Composition pharmaceutique pour traiter ou prévenir la céphalée, la migraine ou les céphalées vasculaires de Horton comprenant une quantité thérapeutiquement efficace de BIBN4096BS ou d'un sel physiologiquement acceptable de celle-ci et un médicament anticéphalée (A) choisi parmi le sumatriptan, le zolmitriptan et la dihydroergotamine ou un sel physiologiquement acceptable de ceux-ci sous forme d'une préparation combinée pour l'administration simultanée ou successive.

2. Composition pharmaceutique selon la revendication 1 comprenant une unité de prise unique de 0,1 à 10 mg de BIBN4096BS et
une unité de prise unique de 1 à 100 mg de sumatriptan ou
une unité de prise unique de 0,1 à 2,5 mg de zolmitriptan ou
une unité de prise unique de 0,1 à 5 mg de dihydroergotamine.

3. Kit de parties pour traiter ou prévenir la céphalée, la migraine ou les céphalées vasculaires de Horton, lequel kit comprend

   (a) une première rétention contenant une composition pharmaceutique comprenant une quantité thérapeutiquement efficace de BIBN4096BS ou d'un sel physiologiquement acceptable de celle-ci et un ou plusieurs diluants et/ou supports pharmaceutiquement acceptables ; et
   (b) une seconde rétention contenant une composition pharmaceutique comprenant du sumatriptan, du zolmitriptan ou de la dihydroergotamine ou un sel physiologiquement acceptable de ceux-ci et un ou plusieurs diluants et/ou supports pharmaceutiquement acceptables.

4. Kit de parties selon la revendication 3, lequel kit comprend du sumatriptan ou un sel physiologiquement acceptable de celui-ci dans la seconde rétention.

5. Utilisation de BIBN4096BS ou d'un sel physiologiquement acceptable de celle-ci en combinaison avec du sumatriptan, du zolmitriptan ou de la dihydroergotamine ou un sel physiologiquement acceptable de ceux-ci pour la production d'une composition pharmaceutique pour traiter ou prévenir la céphalée, la migraine ou les céphalées vasculaires de Horton.

6. Utilisation selon la revendication 5 **caractérisée en ce que** BIBN4096BS, ou un sel physiologiquement acceptable de celle-ci, est destinée à être administrée par voie intraveineuse ou sous-cutanée en une posologie de 0,0001 à 3 mg/kg de poids corporel ou par voie orale, nasale ou par inhalation en une posologie de 0,1 à 10 mg/kg de poids corporel, une, deux ou trois fois par jour et
le sumatriptan ou un sel physiologiquement acceptable de celui-ci est administré par voie orale en une posologie de 0,03 à 1,43 mg/kg de poids corporel une, deux ou trois fois par jour ou
par voie intraveineuse ou sous-cutanée en une posologie de 0,002 à 0,09 mg/kg de poids corporel une ou deux fois par jour ou
par voie rectale en une posologie de 0,007 à 0,36 mg/kg de poids corporel une ou deux fois par jour ou
par voie nasale en une posologie de 0,006 à 0,29 mg/kg de poids corporel une ou deux fois par jour ou
le zolmitriptan ou un sel physiologiquement acceptable de celui-ci est destiné à être administré par voie orale en une posologie de 0,0007 à 0,036 mg/kg de poids corporel une ou deux fois par jour ou
la dihydroergotamine ou un sel physiologiquement acceptable de celle-ci est destinée à être administrée par voie orale en une posologie de 0,001 à 0,07 mg/kg de poids corporel une ou deux fois par jour.

7. Utilisation de BIBN4096BS ou d'un sel physiologiquement acceptable de celle-ci pour la production d'une composition pharmaceutique ou d'un kit de parties selon l'une quelconque des revendications 1 à 4.